# EUROPEAN PATENT SPECIFICATION

(11) **EP 1 948 027 B1**
(45) Date of publication and mention of the grant of the patent: **09.04.2014**
(21) Application number: 06789351.1
(22) Date of filing: 02.08.2006
(51) Int. Cl.: A61B 10/00, A61B 10/06, A61B 17/34, A61B 18/14, A61B 18/20, A61B 17/10, A61B 17/12, A61B 17/22

(54) **TISSUE CUTTING DEVICES HAVING HEMOSTASIS CAPABILITY**
GEWEBESCHNEIDEVORRICHTUNGEN MIT HÄMOSTASE-FÄHIGKEIT
DISPOSITIFS DE DÉCOUPE DE TISSU AUX PROPRIÉTÉS HÉMOSTATIQUES

(30) Priority: 16.09.2005 US 227284
(43) Date of publication of application: 30.07.2008
(73) Proprietor: Boston Scientific Limited, Christ Church (BB)
(72) Inventor: GEITZ, Kurt, A., E., Sudbury, Massachusetts 01776 (US); HOFFMAN, David, Concord, Massachusetts 01742 (US)
(74) Representative: Hauck Patent- und Rechtsanwälte
(86) International application number: PCT/US2006/030343
(87) International publication number: WO 2007/040800

(56) References cited:
- WO-A-99/53851
- WO-A2-02/094341
- WO-A2-2004/052171
- US-A- 5 226 908
- US-A1- 2001 049 509
- US-A1- 2002 049 442
- US-A1- 2004 181 242
- US-B1- 6 352 503

## Description

### DESCRIPTION OF THE INVENTION

### Field of the Invention

The present invention relates generally to medical devices and related methods. More specifically, particular embodiments of the invention relate to tissue acquisition devices (e.g., biopsy forceps) having a separate hemostasis capability for use in, for example, acquiring tissue samples from a body.

### Description of Related Art

Biopsy forceps are widely used to obtain tissue samples. A biopsy forceps generally includes a distal cutting member configured to sever tissue samples and a tissue receptacle for holding one or more of the severed tissue samples. Severing tissue from a tissue site causes that tissue site to bleed. To stop or minimize the bleeding during the procedure, "hot" biopsy forceps may be used to direct cauterizing electric current or thermal energy to the tissue site through the distal cutting member to cauterize the bleeding tissue site. Such "hot" biopsy forceps, however, may damage the tissue samples collected in the biopsy forceps, such that a proper diagnosis of the tissue samples may be difficult.

Thus, there is a need for biopsy forceps having a separate hemostasis capability, which does not damage the tissue sample without compromising the tissue acquisition and/or hemostasis capabilities. US-A-2002/0049442 discloses a medical device having the features of the preamble of claim 1.

### SUMMARY OF THE INVENTION

Therefore, various exemplary embodiments of the invention may provide biopsy forceps having a separate hemostatic device integrated with the biopsy forceps, which may effectively stop the bleeding at the tissue site without damaging the tissue samples obtained by the biopsy forceps.

To attain the advantages and in accordance with the purpose of the invention, as embodied and broadly described herein, one aspect of the invention may provide a medical device comprising an elongated tubular member having a proximal end and a distal end, a tissue cutting member proximate the distal end of the tubular member, and a hemostatic member proximate the distal end of the tubular member and adjacent the tissue cutting member, as claimed in claim 1.

Preferred embodiments are disclosed in the dependent claims.

In some aspects of the invention, the tissue cutting member may comprise a tissue acquisition device. The tissue acquisition device may comprise a biopsy forceps. Alternatively or additionally, the tissue cutting member may comprise at least one of a snare, scissors, pincer, needle, knife, needleknife, and basket.

In another aspect, the hemostatic member may comprise one of a laser fiber probe, a heater probe, an injection needle configured to inject a therapeutic agent, an electrocautery device, a clip applicator, a ligation device configured to apply a band or loop, and a gas-induced plasma probe.

According to one aspect of the invention, the elongated member may be sufficiently flexible to traverse tortuous anatomy of a patient.

Additional objects and advantages of the invention will be set forth in part in the description which follows, and in part will be obvious from the description, or may be learned by practice of the invention. The objects and advantages of the invention will be realized and attained by means of the elements and combinations particularly pointed out in the appended claims.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this specification, illustrate an exemplary embodiment of the invention and together with the description, serve to explain the principles of the invention. In the drawing:
Fig. 1 is a schematic view of a biopsy forceps having separate hemostasis capability, according to an exemplary embodiment of the invention;
Fig. 2 is a partial schematic view of the distal portion of the biopsy forceps shown in Fig. 1;
Figs. 3-5 are schematic cross-sectional views of the biopsy forceps shown in Fig. 2, along III-III plane, according to various exemplary embodiments of the invention;
Fig. 6 is a partial schematic view of a hemostatic device, according to another exemplary embodiment of the invention;
Fig. 7 is a partial schematic view of a hemostatic device; according to still another exemplary embodiment of the invention;
Fig. 8 is a partial schematic view of a hemostatic device, according to still yet another exemplary embodiment of the invention; and
Figs. 9-11 are schematics illustrating an exemplary method of a surgical procedure using the device of Fig. 1, according to an exemplary embodiment of the invention.

### DESCRIPTION OF THE EMBODIMENTS

Reference will now be made in detail to the exemplary embodiments consistent with the present invention, examples of which are illustrated in the accompanying drawings. Wherever possible, the same reference numbers will be used throughout the drawings to refer to the same or like parts.

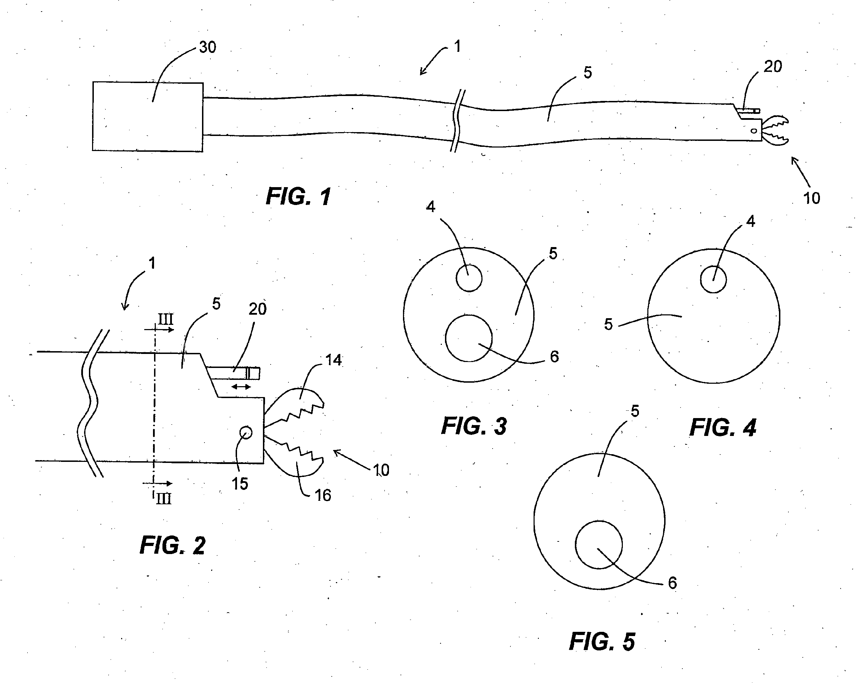

Fig. 1 illustrates an endoscopic medical device 1 (e.g., tissue acquisition device) having a separate hemostatic device for use in, for example, obtaining tissue samples from a patient's body, according to an exemplary embodiment of the invention. While the invention will be described in connection with a particular tissue acquisition device (i.e., biopsy forceps), embodiments of the invention may be used with any other types of tissue acquisition device (e.g., snares, scissors, needles, pincers, knives, knifeneedles, or baskets). Moreover, certain aspects of the inventions may be applied to, or used in connection with, other numerous surgical applications involving tissue cutting that may require a hemostatic treatment.

As shown in Fig. 1, the medical device 1 may comprise a generally tubular body 5 or shaft, a biopsy jaw assembly 10 coupled to the distal portion of the tubular body 5, and a hemostatic device 20 adjacent the jaw assembly 10 in the distal portion of the tubular body 5. The tubular body 5 may be coupled to a suitable actuation member 30 located at the proximal end of the tubular body 5. The actuation member 30 may be connected to the jaw assembly 10 and/or the hemostatic device 20 via a suitable control mechanism, such as, for example, one or more control wires (not shown). In some exemplary embodiments, a separate actuation member may be provided for each of the jaw assembly 10 and the hemostatic device 20.

As best shown in Fig. 2, the jaw assembly 10 may include a pair of jaws 14, 16 having sharp cutting edges or teeth for severing tissue. Actuation of the actuation member may cause the pair of jaws 14, 16 to open and close to sever tissue. In some exemplary embodiments, as shown in Fig. 2, the pair of jaws 14, 16 may be pivotally coupled to the tubular body 5 via a suitable pivot member 15. The jaw assembly 10 and its corresponding actuation member and/or control mechanism may be of any conventional type known in the art.

The hemostatic device 20 may comprise a laser fiber configured to direct a localized laser energy onto the bleeding tissue site, so as to coagulate the bleeding tissue. Alternatively or additionally, the hemostatic device 20 may comprise an applicator, an injection needle, or a spray nozzle configured to apply a therapeutic agent, such as, for example, hemostatic agent, sclerosing agent, and/or coagulating agent, to the surface of the bleeding tissue site. For example, in some exemplary embodiments, epinephrine, ethanol, and hypertonic saline may be used for non-variceal bleeding, and sodium tetradecyl sulfate, sodium morrhuate, ethanolamine oleate, polidocanol, and ethanol may be used for variceal bleeding. Any other known therapeutic agent may be used alternatively or additionally

The hemostatic device 20 is axially extendable and may be radially rotatable to facilitate accurate positioning of the device 20 with respect to the tissue to be treated.

Depending on the type of the hemostatic device 20, the jaw assembly 10 may be suitably insulated from the hemostatic device 20 to prevent or minimize the damage to the severed tissue contained in the biopsy forceps.

The tubular body 5 may be sufficiently flexible to traverse through a tortuous body lumen, yet sufficiently stiff to resist compressive force applied thereto during normal operation. The tubular body 5 may or may not be hollow. For example, the tubular body 5 may define at least one lumen 4, 6 along its longitudinal axis to accommodate the jaw assembly 10 and/or the hemostatic device 20, as shown in Fig. 3. In this particular example not according to the invention, at least one of the jaw assembly 10 and the hemostatic device 20 may be separately provided so as permit independent movement in and out of the lumen 4, 6 relative to each other. In an embodiment; the tubular body 5 may be an endoscope defining a plurality of working lumens 4, 6, through which the jaw assembly 10 and/or the hemostatic device 20 may pass.

The jaw assembly 10 may be fixedly connected to the tubular body 5, for example by a pivot pin 15 as shown in Figs. 1 and 2, and the hemostatic device 20 may be movably arranged within the lumen 4 of the tubular body 5, such as the tubular body 5 shown in Fig. 4. When the movable device is not in use, the movable device may be removed from the lumen 4, 6, so that other suitable devices may be inserted into the lumen to reach the tissue site, if desired.

According to an example not according to the invention, both the jaw assembly 10 and the hemostatic device 20 may be fixedly connected to the tubular body 5. For example, the jaw assembly 10 and the hemostatic device 20 may be integrally formed with the tubular body 5. When the jaw assembly 10 and the hemostatic device 20 are integrally formed, the tubular body 5 may still define one or more lumens or passageways therein.

With the jaw assembly 10 fixedly connected to the tubular body 5, the hemostatic device 20 is configured to extend radially and axially, at least to a certain extent, to facilitate positioning relative to the tissue site. This may permit accurate positioning of the jaw assembly 10 and/or the hemostatic device 20 with respect to the target tissue.

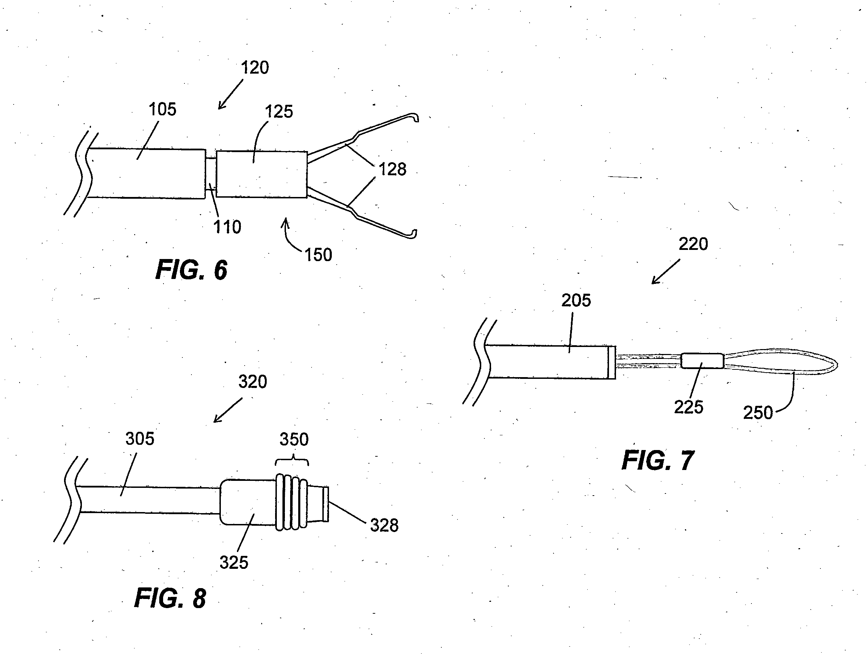

According to some aspects of the invention, the medical device 1 may employ various different types of a hemostatic device. For example, the device 1 may include a clip applicator 120 configured to deploy one or more detachable clips (e.g., hemoclips), as shown in Fig. 6. The applicator 120 may comprise an elongated tube 105 extending from a suitable actuation handle (not shown) and one or more clips 150 disposed proximate the distal end of the tube 105. In some exemplary embodiments, the clip 150 may be detachably coupled to the distal end of the tube 105 via a suitable connecting member 110. The clip 150 may comprise a main body 125 and a pair of jaws 128 pivotally coupled to the main body 125. In an exemplary embodiment, the jaws 128 may have more than two jaws. The jaws 128 may be opened or closed by actuation of the actuation handle. The actuation handle may also cause detachment and/or deployment of the clip 150 from tube 105. The clip 150 may be configured to be re-coupled to the tube 105 so as to permit repositioning or removal of the clip 150.

In another exemplary embodiment, the device 1 may include a loop applicator 220. As shown in Fig. 7, the loop applicator 220 may comprise an elongated tube 205 and a ligation loop 250 detachably disposed proximate the distal end of the tube 205. The proximal end of the elongated tube 205 may be coupled a suitable actuator (not shown) to control the movement of the loop 250. The loop 205 may include a slidable sheath 225 so as to adjust the size and the ligating tension of the loop 250. The loop 250 may be used to ligate a target tissue prior to or after a medical procedure. For example, during a polypectomy procedure, the loop 250 may be placed around a target tissue below a location to be cut, so as to minimize a post-procedure bleeding.

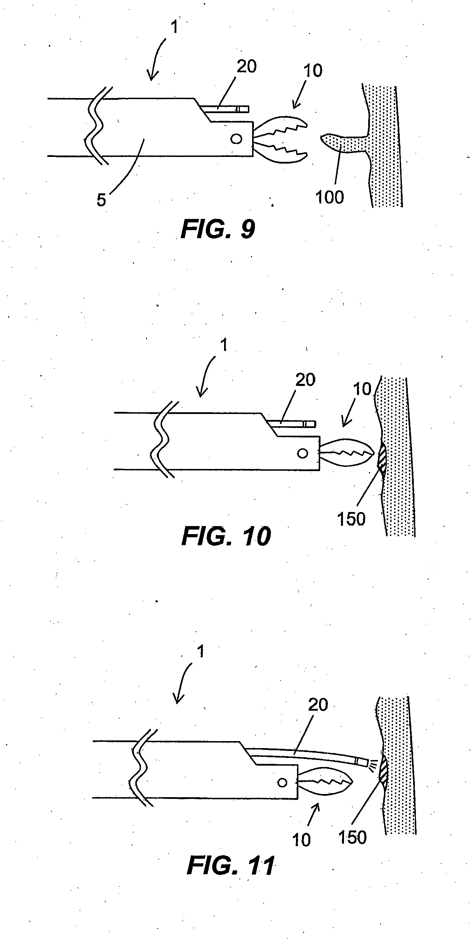

According to still another exemplary embodiment of the invention, the device 1 may include a band applicator 320. As shown in Fig. 9, the applicator 320 may include an elongated member 305 extending from a suitable actuation handle (not shown) and a distal member 325 coupled to the distal end of the elongated member 305. The distal member 325 may include one or more bands 350 or rings that may be placed onto a target tissue to ligate the tissue. The elongated member 305 may define a hollow lumen therethrough, and the distal member 325 may define an opening 328 that may communicate with the hollow lumen of the elongated member 305. The hollow lumen of the elongated member 305 may extend to the actuation handle and connect to a suitable suction source. To place the bands 350 onto a target tissue, according to an exemplary embodiment, the suction source may be turned on and a target tissue may be held by, or suctioned into, the opening 328. Once the target tissue is secured to the opening 328, the band 350 may be placed over the target tissue to ligate the tissue.

Other various types of a hemostatic device may be used alternatively or additionally. For example, in some exemplary embodiments, the hemostatic device 20 may comprise a gas-induced plasma coagulation device (e.g., argon plasma coagulation device) configured to direct heat energy generate by ionizing the gas onto the bleeding tissue site to coagulate the bleeding tissue. Any other hemostasis device known in the art may also be used additionally or alternatively.

The operation of the medical device 1, according to an exemplary embodiment of the invention, will be described in detail with reference to Figs. 9-11. While operational aspects of the invention will be described in connection with a particular tissue removal procedure, embodiments of the invention may be applied to other suitable medical procedures, or used in connection with any other suitable medical devices, without departing from the scope of the invention.

As shown in Fig. 9, the device 1 may be inserted into a body lumen of a patient, for example over a suitable guidewire, through a lumen of an endoscope, or via any other conventional method, to position its distal end near the tissue 100 to be removed. Proper positioning of the device 1 may be assisted by any suitable, known visualization method. Once the distal end of the device 1 is properly positioned, the actuation member 30 may be actuated to operate the jaw assembly 10 and to sever the tissue 100 from the target site, as shown in Fig. 10. Severing of the tissue 100 may cause bleeding at the tissue site 150 where the severing occurred. The hemostatic device 20 may be used to stop the bleeding at the tissue site 150, as shown in Fig. 11. It should be understood that any other hemostatic device described above may be used instead. If desired, the hemostatic device 20 may be extended and/or rotated to obtain proper positioning of the device 20 with respect to the bleeding tissue site 150. Once the bleeding stops, the device 1 may be removed from the patient's body or moved to another location to repeat the procedure on another tissue. In certain applications, if desired, one or both of the jaw assembly 10 and the hemostatic device 20 may be removed from the tubular member 5 and replaced with other suitable devices, with the tubular member 5 remaining in place within the patient's body.

Other embodiments of the invention will be apparent to those skilled in the art from consideration of the specification and practice of the invention disclosed herein. It is intended that the specification and examples be considered as exemplary only, with a true scope of the invention being indicated by the following claims.

## Claims

1. A medical device (1) for insertion in a lumen of an endoscope comprising:
an elongated tubular member (5) sized to fit through the lumen of an endoscope having a proximal end, a distal end and a lumen (4);
a tissue cutting member (10) proximate the distal end of the tubular member (5); and
a hemostatic member (20) proximate the distal end of the tubular member (5) and adjacent the tissue cutting member (10), wherein the tissue cutting member (10) is fixedly connected to the tubular member (5) **characterized in that** the hemostatic member (20) is movably arranged within the lumen (4) of the tubular member (5) and configured to extend axially and radially to facilitate positioning relative to the tissue site.

2. The device (1) of claim 1, wherein the tubular member (5) defines a lumen (4, 6) extending between the proximal end and the distal end.

3. The device (1) of claim 1, wherein the tissue cutting member (10) comprises a tissue acquisition device.

4. The device (1) of claim 3, wherein the tissue acquisition device comprises a biopsy forceps.

5. The device (1) of claim 1, wherein the tissue cutting member (10) comprises at least one of a snare, scissors, pincer, needle, knife, needleknife, and basket.

6. The device (1) of claim 1, wherein the hemostatic member (20) comprises one of laser fiber probe, a heater probe, an injection needle configured to inject a therapeutic agent, an electrocautery device, a clip applicator, a ligation device configured to apply a band or a loop, and a gas induced plasma probe.

7. The device (1) of claim 1, wherein the elongated member (5) is sufficiently flexible to traverse tortuous anatomy of a patient.

8. The device (1) of claim 1, further including an actuation member (30) located at the proximal end of the tubular member (5), the actuation member (30 being connected to both the tissue cutting member (10) and the hemostatic member (20).

9. The device (1) of claim 1, wherein the hemostatic member (20) and the tissue cutting member (10) both extend distally from the distal end of the tubular member.

10. The device (1) of claim 1, wherein the tissue cutting member (10) includes jaws (14, 16) pivotally coupled to the tubular member (5).

11. The device (1) of claim 10, wherein the jaws (14, 16) are pivotally coupled to the tubular member (5) by a pivot pin (15).

12. The device (1) of claim 1, wherein the hemostatic member (20) comprises a clip applicator (120) attached to the elongated member (105).

13. The dcvice (1) of claim 1, wherein the hemostatic member (20) comprises a ligation device (220; 320) attached to the elongated member (205; 305) and configured to apply a band (350) or loop (250).

## Patentansprüche

1. Medizinische Vorrichtung (1) zur Einführung in ein Lumen eines Endoskopes, umfassend:
ein längliches röhrenförmiges Element (5), dessen Größe dazu ausgelegt ist, durch das Lumen eines Endoskopes mit einem proximalen Ende, einem distalen Ende und einem Lumen (4) zu passen;
ein zu dem distalen Ende des röhrenförmigen Elementes (5) proximales Gewebeschneidelement (10), und
ein hämostatisches Element (20), das proximal zu dem distalen Ende des röhrenförmigen Elementes (5) und angrenzend an das Gewebeschneidelement (10) ist, wobei das Gewebeschneidelement (10) fest mit dem röhrenförmigen Element (5) verbunden ist, **dadurch gekennzeichnet, dass** das hämostatische Element (20) verschieblich innerhalb des Lumens (4) des röhrenförmigen Elementes (5) angeordnet und dazu konfiguriert ist, axial und radial verfahrbar zu sein, um die Positionierung in Bezug auf die Gewebestelle zu erleichtern.

2. Vorrichtung (1) gemäß Anspruch 1, wobei das röhrenförmige Element (5) ein sich zwischen dem proximalen Ende und dem distalen Ende erstreckendes Lumen (4, 6) definiert.

3. Vorrichtung (1) gemäß Anspruch 1, wobei das Gewebeschneidelement (10) eine Vorrichtung zum Erfassen von Gewebe aufweist.

4. Vorrichtung (1) gemäß Anspruch 3, wobei die Vorrichtung zum Erfassen von Gewebe eine Biopsiezange umfasst.

5. Vorrichtung (1) gemäß Anspruch 1, wobei das Gewebeschneidelement (10) mindestens eines aus einer Schlinge, Schere, Zange, Nadel, Messer, Nadelmesser und einem Korb umfasst.

6. Vorrichtung (1) gemäß Anspruch 1, wobei das hämostatische Element (20) eines aus einer Glasfaser-Lasersonde, einer Heizsonde, einer zum Injizieren eines therapeutischen Mittels konfigurierten Injektionsnadel, einer Elektrokauterisierungsvorrichtung, einem Klammerapplikator, einer zum Anbringen eines Bandes oder einer Schleife konfigurierten Ligaturvorrichtung und einer gasinduzierten Plasmasonde umfasst.

7. Vorrichtung (1) gemäß Anspruch 1, wobei das längliche Element (5) ausreichend flexibel zum Durchqueren von gewundener Anatomie eines Patienten ist.

8. Vorrichtung (1) gemäß Anspruch 1, ferner beinhaltend ein an dem proximalen Ende des röhrenförmigen Elementes (5) befindliches Betätigungselement (30), wobei das Betätigungselement (30) sowohl mit dem Gewebeschneidelement (10) als auch mit dem hämostatischen Element (20) verbunden ist.

9. Vorrichtung (1) gemäß Anspruch 1, wobei das hämostatische Element (20) und auch das Gewebeschneidelement (10) sich distal von dem distalen Ende des röhrenförmigen Elementes erstrecken.

10. Vorrichtung (1) gemäß Anspruch 1, wobei das Gewebeschneidelement (10) schwenkbar mit dem röhrenförmigen Element (5) gekoppelte Klemmbacken (14, 16) beinhaltet.

11. Vorrichtung (1) gemäß Anspruch 10, wobei die Klemmbacken (14, 16) mittels eines Gelenkstiftes (15) schwenkbar mit dem röhrenförmigen Element (5) gekoppelt sind.

12. Vorrichtung (1) gemäß Anspruch 1, wobei das hämostatische Element (20) einen an dem länglichen Element (105) befestigten Klammerapplikator (120) umfasst.

13. Vorrichtung (1) gemäß Anspruch 1, wobei das hämostatische Element (20) eine an dem röhrenförmigen Element (205; 305) befestigte Ligaturvorrichtung (220; 320) umfasst, die dazu konfiguriert ist, ein Band (350) oder eine Schleife (250) zu applizieren.

## Revendications

1. Dispositif médical (1) pour l'insertion dans la lumière d'un endoscope, comportant:
un élément tubulaire allongé (5), dimensionné pour s'adapter à franchir la lumière d'un endoscope qui a une extrémité proximale, une extrémité distale et une lumière (4);
un élément de découpe de tissu (10), proximal à l'extrémité distale de l'élément tubulaire (5); et
un élément hémostatique (20), proximal à l'extrémité de l'élément tubulaire (5) et adjacent à l'élément de découpe de tissu (10), l'élément de découpe de tissu (10) étant fixement relié à l'élément tubulaire (5), **caractérisé en ce que** l'élément hémostatique (20) est arrangé de manière mobile au-dedans de la lumière (4) de l'élément tubulaire (5) et configuré pour être radialement et axialement extensible afin de faciliter le positionnement par rapport au site du tissu.

2. Dispositif selon la revendication 1, dans lequel l'élément tubulaire (5) définit une lumière (4, 6) qui s'étend entre l'extrémité proximale et l'extrémité distale.

3. Dispositif selon la revendication 1, dans lequel l'élément de découpe de tissu (10) comporte un dispositif d'acquisition de tissu.

4. Dispositif selon la revendication 3, dans lequel le dispositif d'acquisition de tissu comporte des pinces à biopsie.

5. Dispositif selon la revendication 1, dans lequel l'élément de découpe de tissu (10) comporte au moins un entre un trappeur, des ciseaux, une pince, une aiguille, un couteau, une aiguille-couteau et une cage.

6. Dispositif selon la revendication 1, dans lequel l'élément hémostatique (20) comporte un élément parmi une sonde laser à fibre, une sonde à chauffage, une seringue d'injection configurée à l'injection d'un agent thérapeutique, un dispositif de cautérisation, un applicateur de pinces, un dispositif de ligature configuré à appliquer une bande ou un boucle, et une sonde plasma induite par gaz.

7. Dispositif selon la revendication 1, dans lequel l'élément tubulaire allongé (5) est suffisamment flexible pour traverser l'anatomie tortueuse d'un patient.

8. Dispositif selon la revendication 1, comportant en outre un élément d'actionnement (30) situé dans l'extrémité proximale de l'élément tubulaire (5), l'élément d'actionnement (30) étant relie et à l'élément de découpe de tissu (10) et à l'élément hémostatique (20).

9. Dispositif selon la revendication 1, dans lequel et l'élément hémostatique (20) et l'élément de découpe de tissu (10) s'étendent distalement de l'extrémité distale de l'élément tubulaire.

10. Dispositif selon la revendication 1, dans lequel l'élément de découpe de tissu (10) inclût des mors (14, 16) accouplés à l'élément tubulaire (5) de manière pivotante.

11. Dispositif selon la revendication 10, dans lequel les mors (14, 16) sont accouplés à l'élément tubulaire (5) de manière pivotante par un pivot.

12. Dispositif selon la revendication 1, dans lequel l'élément hémostatique (20) comporte un applicateur de pinces (120) attaché à l'élément allongé (105).

13. Dispositif selon la revendication 1, dans lequel l'élément hémostatique (20) comporte un dispositif de ligature (220; 320) attaché à l'élément tubulaire (205; 305) et configuré à appliquer un bande (350) ou un boucle (250).
